# EUROPÄISCHE PATENTSCHRIFT

(11) **EP 2 063 371 B1**
(45) Veröffentlichungstag und Bekanntmachung des Hinweises auf die Patenterteilung: **04.06.2014**
(21) Anmeldenummer: 07022002.5
(22) Anmeldetag: 13.11.2007
(51) Int. Cl.: G06F 19/00

(54) **Vorrichtung und Verfahren zur Dokumentation medizinischer Daten**
Device and method for documenting medical data
Dispositif et procédé de documentation de données médicales

(43) Veröffentlichungstag der Anmeldung: 27.05.2009
(73) Patentinhaber: How to Organize (H2O) GmbH, 10115 Berlin (DE)
(72) Erfinder: Abri, Omid Prof. Dr., 14129 Berlin (DE)
(74) Vertreter: Fugmann, Winfried

(56) Entgegenhaltungen:
- WO-A-2007/073420
- FR-A- 2 856 222
- US-A- 5 654 750
- US-A1- 2003 108 327
- US-A1- 2003 159 141
- GUERLAIN S ET AL: "Assessing team performance in the operating room: Development and use of a ''black-box'' recorder and other tools for the intraoperative environment" JOURNAL OF THE AMERICAN COLLEGE OF SURGEONS, COLLEGE, CHICAGO, IL, US, Bd. 200, Nr. 1, 1. Januar 2005 (2005-01-01), Seiten 29-37, XP004699269 ISSN: 1072-7515

## Beschreibung

Die vorliegende Erfindung betrifft eine Vorrichtung zur Dokumentation medizinischer Daten nach dem Oberbegriff von Anspruch 1 sowie ein Verfahren zur Dokumentation medizinischer Daten.

Derartige Vorrichtungen und Verfahren sind bekannt. So sind beispielsweise endoskopische Bilddokumentationssysteme bekannt, mit denen bestimmte Bilddaten zusammen mit entsprechenden Patienteninformationen aufgenommen, bearbeitet und gespeichert werden können. Ein solches System wird beispielsweise unter dem Namen KARL STORZ AIDA® in verschiedenen Ausführungen angeboten.

Ein solches Bilddokumentationssystem kann über eine Schnittstelle mit einer zentralen busgestützten OP-Steuerung verbunden sein. Eine solche OP-Steuerung und ein solches Bussystem sind beispielsweise in EP 1 034 480 B1 und in WO 02/19957 A2 beschrieben. Es ist auch bekannt, über einen Datenlogger intraoperative Geräte- bzw. Funktionsparameter auf einer zentralen Steuereinheit aufzuzeichnen.

Weiterhin sind aus DE 10 2005 025 903 A1 und aus US 7,231,135 B2 Systeme zur Speicherung und Bearbeitung medizinischer Daten, nämlich medizinischer Videodaten bekannt.

Aus WO 2007/073420 A1 ist es bekannt, digitale Videodaten von einer Mehrzahl von Videoquellen in einer Mehrzahl von Operationssälen über ein Netzwerk zu übertragen, so dass Benutzer von einem beliebigen Ort des Netzwerks die Videodaten betrachten können. Dabei ist auch eine Datenspeicherung vorgesehen. US 5,654,750 offenbart ein System zur automatischen Aufzeichnung jedes chirurgischen Eingriffs in einem Operationssaal, wobei Bewegungs- und photoelektrische Sensoren im Operationssaal angebracht sind, um die Anwesenheit von Personen zu detektieren. Die Aufzeichnung beginnt automatisch, wenn eine beliebige Aktivität im Operationssaal detektiert wird, und wird bis zur vollständigen Beendigung der Aktivitäten weitergeführt.

Würde bei den bekannten Systemen zur Dokumentation medizinischer Daten eine fortlaufende Aufzeichnung, insbesondere eine forlaufende Videoaufzeichnung aller Aktivitäten im Operationssaal (OP) erfolgen, so würden nach relativ kurzer Zeit die Speichermedien überlaufen und keine weitere Speicherung mehr erfolgen können. Andererseits wäre es für eine retrospektive Qualitätskontrolle im OP wünschenswert, auf eine vollständige Videoaufzeichnung zurückgreifen zu können.

Es ist daher die Aufgabe der vorliegenden Erfindung, eine Vorrichtung und ein Verfahren anzugeben, mit dem eine fortlaufende Speicherung von medizinischen Daten, insbesondere Videodaten und/oder Sprachdaten aus einem OP, möglich ist, ohne dass die verfügbaren Speichermedien nach kurzer Zeit erschöpft sind.

Diese Aufgabe wird durch eine Vorrichtung gemäß Anspruch 1 und ein System nach Anspruch 14 sowie durch ein Verfahren gemäß Anspruch 17 gelöst.

Dadurch, dass eine Speichereinrichtung vorgesehen ist, die einen ersten Datenspeicher enthält, der als Ringspeicher ausgebildet ist, auf dem die dort gespeicherten Daten jeweils nach einer vorbestimmten Zeit, nachdem sie eingespeichert wurden, wieder gelöscht werden, ist sichergestellt, dass das Speichermedium nicht erschöpft wird, sondern stets zur Aufnahme neuer Daten zur Verfügung steht. Dabei kann das Löschen auch durch Überschreiben der Daten geschehen.

Darüber hinaus sind mindestens eine Datenübernahmeeinrichtung zur Übernahme von Daten von mindestens einer Datenquelle, mindestens eine Speichereinrichtung zur Speicherung von Daten, mindestens eine Anzeigeeinrichtung zur Anzeige von Daten für einen Benutzer, und mindestens eine Eingabeeinrichtung zur Eingabe von Daten und/oder Anweisungen durch den Benutzer vorgesehen. Die Datenübernahmeeinrichtung kann zur Übernahme von Daten beispielsweise über ein Netzwerk oder auch von einem Datenträger, wie einer CD oder DVD, ausgebildet sein. Als weitere Datenquellen kommen insbesondere Videokameras, beispielsweise endoskopische Kameras oder auch Raumkameras, andere Datenerfassungseinrichtungen, wie beispielsweise Mikrofone, sowie die Datenübernahme von drahtlos lesbaren Datenträgern, wie beispielsweise RFID-Systemen, in Frage. Für einen Benutzer steht eine Anzeigeeinrichtung, insbesondere ein Computerbildschirm oder andere Datenausgabeeinrichtungen wie etwa Lautsprecher, und zur Eingabe von Daten und Anweisungen eine Eingabeeinrichtung, beispielsweise eine Tastatur, ein Touch Screen, oder eine Sprachsteuerung zur Verfügung.

Ist aufgrund beispielsweise einer nach einer Operation aufgetretenen Komplikation ein Zugriff auf die bei dieser Operation aufgenommenen Daten notwendig, so kann der jeweils relevante Teil der Daten dem Ringspeicher entnommen und in einem anderen Speicher zur weiteren Verwertung gespeichert werden oder auf dem Ringspeicher wird keine weitere Datenspeicherung und damit auch keine weitere Datenlöschung vorgenommen, so dass die Daten zur Auswertung zur Verfügung stehen. Es kann daher auch ein weiterer Ringspeicher vorgesehen sein, der dann für die fortlaufende Datenspeicherung zur Verfügung steht.

Gemäß einer bevorzugten Ausführungsform enthält die Speichereinrichtung einen zweiten Datenspeicher, der nicht als Ringspeicher ausgebildet ist. Dies hat den Vorteil, dass einige Daten, beispielsweise solche, die nur wenig Speicherplatz erfordern oder die langfristig gespeichert werden sollen, nicht von der regelmäßigen Löschung auf dem Ringspeicher betroffen sind.

Gemäß einer weiteren bevorzugten Ausführungsform kann der Benutzer die Übernahme von Daten in den ersten oder in den zweiten Datenspeicher steuern, d. h., der Benutzer kann angeben, welche Daten langfristig gespeichert werden sollen und welche nach einer vorbestimmten Zeit wieder gelöscht werden können. Dies kann, ebenso wie die Festlegung der vorbestimmten Zeit, für jeden Benutzer unterschiedlich geschehen, wobei jeweils eine Identifikation oder Autorisierung des Benutzers erforderlich gemacht werden kann. Damit ist je nach Art der Daten, beispielsweise für unterschiedliche Arten von Operationen, eine jeweils angepasste Festlegung und damit eine optimale Ausnutzung des Speicherplatzes möglich.

Gemäß einer weiteren bevorzugten Ausführungsform erfolgt die Übernahme von Daten in den ersten oder in den zweiten Datenspeicher zumindest teilweise aufgrund von vorbestimmten Kriterien, die nicht vom Benutzer beeinflussbar sind. Auch die Zeit, nach der die im Ringspeicher gespeicherten Daten automatisch gelöscht werden, kann benutzerunabhängig vorgegeben sein. Hierdurch ist die Erfüllung von Qualitätssicherungs- oder gesetzlichen Erfordernissen sicherer zu gewährleisten. Eine geeignete Zeitspanne, nach der die gespeicherten Daten, wenn hierauf kein Zugriff erfolgt ist, gelöscht werden können, ist beispielsweise ca. 4 Wochen, da sich in der Regel nach dieser Zeit etwaige Komplikationen nach einer Operation herausgestellt hätten.

Gemäß einer besonders bevorzugten Ausführungsform enthalten die gespeicherten Daten Bildinformationen, insbesondere endoskopische Bildinformationen, beispielsweise endoskopische Videobilder. Ebenso können die Videobilder einer Raumkamera, die zumindest den Bereich um den OP-Tisch herum erfasst, gespeichert werden. Dies hat den Vorteil, dass durch die Speicherung von Bilddaten eine besonders lückenlose Dokumentation der Vorgänge bei einer Operation möglich ist, beispielsweise die nachträgliche Erkennung von Fehlern oder Besonderheiten, die dem Operateur während der Operation entgangen waren. Ebenso können auch Sprachdaten erfasst werden, beispielsweise in eine Sprachsteuerung eingegebene Anweisungen, aber auch andere akustische Daten, wie etwa Gespräche oder Geräusche, aus einem OP. Auch dies trägt zu einer besonders vollständigen Dokumentation der Vorgänge bei einer OP bei.

Die erfindungsgemäße Vorrichtung kann insbesondere auch zur Bearbeitung der gespeicherten Daten, insbesondere der Bilddaten, ausgebildet sein. Der Benutzer hat somit die Möglichkeit, Kommentare, Dokumente, Rechercheergebnisse usw. zu den Bilddaten hinzuzufügen, Markierungen in den Bilddaten anzubringen usw.

In einer weiteren besonders bevorzugten Ausführungsform ist die Vorrichtung zur Aufnahme und Speicherung von Daten über die bei einer chirurgischen Operation verwendeten Instrumente und/oder Geräte ausgebildet. Diese Daten können beispielsweise Typ und Serien- oder Inventamummer der betreffenden Instrumente oder Geräte enthalten. Hiermit ist eine vollständigere Dokumentation aller Vorgänge bei der Operation möglich. Ebenso können gleichzeitig Daten des Patienten erfasst werden, insbesondere Anamnesedaten, aber auch beispielsweise Vitalparameter während der Operation.

Gemäß einer weiteren bevorzugten Ausführungsform enthalten die Daten insbesondere Informationen über Ort und/oder Funktion und/oder Parameter der bei der chirurgischen Operation verwendeten Instrumente und/oder Geräte. Die Daten zur Funktion können insbesondere die eingestellten Parameter sein, wie beispielsweise Insufflationsdruck oder HF-Spannung, aber auch damit zusammenhängende Messwerte, wie beispielsweise gemessener Druck, Leckstrom, Temperatur usw. Darüber hinaus kann die Miterfassung von Anästhesiedaten und Vitalparametern möglich sein. So ist eine besonders vollständige Dokumentation der Vorgänge bei einer Operation möglich.

Gemäß einer weiteren besonders bevorzugten Ausführungsform werden die Daten automatisch übernommen. Dies kann sowohl die Übernahme der Bilddaten, wie auch die Übernahme der Instrumenten- und Gerätedaten betreffen. Hierdurch ist sichergestellt, dass unabhängig von der Situation im Operationssaal eine vollständige Dokumentation erfolgt.

Dabei können die Daten insbesondere zumindest teilweise aus einem Netzwerk, von einem Computersystem und/oder von medizinischen Geräten, insbesondere einer endoskopischen Videokamera, aber auch beispielsweise von einer Raumkamera übernommen werden. Insbesondere dann, wenn die erfindungsgemäße Vorrichtung zur Integration in ein busgesteuertes Kommunikationssystem innerhalb des Operationssaals ausgebildet ist, ist dies eine einfache und sichere Lösung, bei der auch alle weiteren operationellen und sicherheitsbedingten Anforderungen innerhalb eines Operationssaals erfüllt werden können. Beispielsweise ist die Einbettung sicherheitsrelevanter Funktionen in ein geschlossenes System möglich, während nicht sicherheitsrelevante Funktionen in einem offenen System ausgeführt werden können.

Gemäß einer weiteren bevorzugten Ausführungsform werden die Daten zumindest teilweise über eine drahtlose Verbindung übernommen. Derartige drahtlose Verbindungen sind in vielen Bereichen gängig, beispielsweise können über ein WLAN Daten innerhalb und außerhalb des OP übertragen werden. Dies hat den Vorteil, dass die Bewegung hindernde Kabelverbindungen entfallen.

Gemäß einer weiteren besonders bevorzugten Ausführungsform umfasst die Vorrichtung weiterhin mindestens eine RFID-Identifikations- und/oder Ortungsvorrichtung. Damit kann ohne weitere Eingaben des Benutzers automatisch festgestellt werden, welche, mit einem entsprechenden Codeträger versehenen Instrumente, Siebe, Geräte usw. bei einer Operation verwendet wurden, ggf. auch, in welcher Konfiguration und zu welcher Zeit.

Erfindungsgemäß wird die Speicherung von Daten im Ringspeicher durch das Vorhandensein eines Patienten im Operationssaal ausgelöst. Damit kann verhindert werden, dass nicht relevante Daten, wie Videobilder oder Sprachdaten aus einem nicht benutzten OP, unnötig Speicherplatz verbrauchen. Andererseits wird zuverlässig sichergestellt, dass dann, wenn die Daten relevant sein könnten, diese, beispielsweise zur Qualitätssicherung, auch gespeichert werden.

Erfindungsgemäß wird das Vorhandensein des Patienten durch ein RFID-System detektiert. Vorteilhafterweise kann auch gleichzeitig eine Identifikation des Patienten oder auch die Übertragung von patientenspezifischen Daten erfolgen. Damit können zur Dokumentation, aber auch intraoperativ dem Operateur sofort die Daten des Patienten zur Verfügung stehen. Hierfür kann insbesondere der Patient oder das Transportmittel des Patienten beispielsweise einen RFID-Codeträger oder ein anderes Identifikationsmittel tragen.

Gemäß einer weiteren bevorzugten Ausführungsform wird dementsprechend die Speicherung von Daten im Ringspeicher automatisch beendet, wenn der Patient den OP verlässt. Auch hierdurch wird sichergestellt, dass nicht unnötig Speicherplatz belegt wird.

Ein erfindungsgemäßes System zur Durchführung medizinischer Eingriffe umfasst eine Einrichtung zur Steuerung und/oder Überwachung mindestens eines Geräts, mindestens ein Gerät zur Verwendung bei einem medizinischen Eingriff, das mit der Steuerungs- und/oder Überwachungseinrichtung verbunden ist, und eine Vorrichtung zur Dokumentation medizinischer Daten nach einem der vorstehenden Ansprüche. Ein solches Gesamtsystem unterstützt in einfacher und sicherer Weise die Ausführung und die automatische Dokumentation aller Vorgänge bei einer Operation.

Hierbei können die für eine vollständige Dokumentation notwendigen Daten insbesondere dann aufgenommen und gespeichert werden, wenn das System weiterhin Mittel zur Identifikation und/oder Ortsbestimmung von medizinischen Instrumenten, medizinischen Geräten und/oder Personen umfasst. Solche Mittel können insbesondere ein RFID-System sein.

Zur Erkennung, ob ein Patient in den OP hineingebracht wird oder den OP verlässt, sind im Eingangsbereich des OP in Bewegungsrichtung hintereinander mehrere Schleusen mit jeweils einem RFID-Lesegerät zum Beispiel an der Eingangstür des OP installieret. Ebenso kann auf diese Weise die Bewegungsrichtung von mit RFID-Codeträgern ausgestatteten Instrumenten, Sieben, Geräten, Personal usw. erkannt werden. Hierdurch kann unabhängig von der Detektierung innerhalb des OP festgestellt werden, welche Personen und/oder Gegenstände sich im OP befinden bzw. zu einer bestimmten Zeit befunden haben.

Ein erfindungsgemäßes Verfahren zur Dokumentation medizinischer Daten umfasst die folgenden Schritte
- Übernahme medizinischer Daten in eine Speichervorrichtung,
- Speichern der medizinischen Daten in einem Ringspeicher, wobei für einen Benutzer ein Zugriff auf die medizinischen Daten möglich ist,
- Löschen und/oder Überschreiben der medizinischen Daten nach einer vorbestimmten Zeit, sofern durch einen Benutzer kein Zugriff erfolgt ist.
Hierdurch wird die erfindungsgemäße Aufgabe einer möglichst vollständigen fortlaufenden Dokumentation medizinischer Eingriffe gelöst.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung.

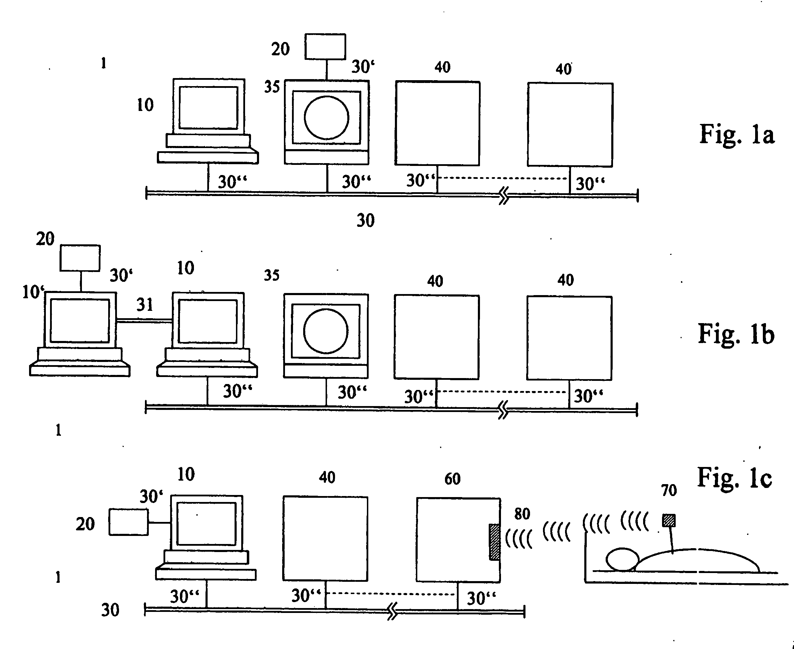

Figur 1 zeigt in schematischer Form bevorzugte Ausführungsbeispiele einer erfindungsgemäßen Vorrichtung.

Gemäß Fig. 1a dient ein Steuerrechner 10 als Eingabevorrichtung zur Eingabe von Anweisungen durch den Benutzer und zur Anzeige von aufgenommenen oder gespeicherten Daten, insbesondere Bilddaten. Der Steuerrechner 10 ist über einen Netzwerkanschluss 30" mit einem Netzwerk 30 verbunden. Über das Netzwerk 30 können Daten ausgetauscht werden. Durch einen weiteren Netzwerkanschluss 30" ist eine endoskopische Kamera 35, die zur Erfassung der mit einem Endoskop gewonnenen Bilddaten dient, mit dem Netzwerk 30 verbunden. Die endoskopische Kamera 35 ist durch die Verbindungsleitung 30', die den Austausch von Daten ermöglicht, an einen Ringspeicher 20 angeschlossen. An das Netzwerk 30 können durch weitere Anschlüsse 30" weitere medizinische Geräte 40 angeschlossen werden. Dies können Geräte in beliebiger Anzahl sein, wie etwa Pumpen, HF-Generatoren, Insufflationseinrichtungen, Absaugeinrichtungen, Beatmungsvorrichtungen usw. Diese können beispielweise über das Netzwerk 30 die Werte der Betriebsparameter übertragen. Darüber hinaus können die Geräte über das Netzwerk 30 beispielweise angesteuert oder mit Strom versorgt werden.

Fig. 1b zeigt eine zweite bevorzugte Ausführungsform der erfindungsgemäßen Vorrichtung. Hierbei ist der Ringspeicher 20 über eine Verbindungsleitung 30' mit einer Eingabevorrichtung 10' in Form eines Personalcomputers verbunden. Der Personalcomputer 10' ist über eine Datenleitung 31 mit der Steuereinrichtung 10 des Netzwerks 30 verbunden. Die beispielweise durch die Kamera 35 erfassten Daten werden durch das Netzwerk 30 zur Steuereinrichtung 10 übertragen, die ihrerseits eine Übertragung der Daten auf den Personalcomputer 10' durch die Datenleitung 31 ermöglicht. Neben dem Ringspeicher 20 verfügt die Dokumentationsvorrichtung 1 in Fig. 1b über weitere Speicherkapazitäten, die jeweils in den Steuereinheiten 10' und 10 angeordnet sind und beispielsweise nach den jeweiligen Erfordernissen verwendet werden können.

Gemäß Fig. 1c kann darüber hinaus eine Empfangsstation 60 vorgesehen sein, die über einen Netzwerkanschluss 30" mit dem Netzwerk 30 verbunden ist. Auf der Empfangstation 60 ist ein Sensor 80, beispielsweise ein Radiosignalempfänger, angeordnet. Der Sensor 80 empfängt die Signale, die ein Transponder beispielsweise in Form einer an einem chirurgischen Instrument oder an einem Patienten angeordnete RFID-Markierung 70 aussendet. Dieses Signal kann beispielweise dazu dienen, dass über das Netzwerk 30 die Speicherung der über das Netzwerk 30 übertragenen Daten im Ringspeicher 20 gestartet wird.

Der übrige Teil der erfindungsgemäßen Vorrichtung gemäß dem Ausführungsbeispiel nach Fig. 1c kann in besonders bevorzugter Weise auch wie in Fig. 1b ausgebildet sein.

## Patentansprüche

1. Vorrichtung zur Dokumentation medizinischer Daten, mit:
- mindestens einer Datenübernahmeeinrichtung zur Übernahme von Daten von mindestens einer Datenquelle,
- mindestens einer Speichereinrichtung zur Speicherung von Daten,
- mindestens einer Anzeigeeinrichtung zur Anzeige von Daten für einen Benutzer, und
- mindestens einer Eingabeeinrichtung zur Eingabe von Daten und/oder Anweisungen durch den Benutzer,
- wobei die Daten Bildinformationen, insbesondere endoskopische Bildinformationen und/oder Sprachdaten aus einem Operationssaal enthalten,
**dadurch gekennzeichnet, dass**
- die Speichereinrichtung einen ersten Datenspeicher enthält, der als Ringspeicher ausgebildet ist,
- so dass auf dem Ringspeicher gespeicherte Daten jeweils nach einer vorbestimmten Zeit gelöscht und/oder überschrieben werden,
- wobei die Speicherung von Daten im Ringspeicher durch das Vorhandensein eines Patienten im Operationssaal ausgelöst wird, wobei das Vorhandensein des Patienten im Operationssaal durch ein RFID-System detektiert wird und wobei in einem Eingangsbereich des Operationssaals hintereinander mehrere Schleusen mit jeweils einem RFID-Lesegerät installiert sind und die Bewegungsrichtung des mit einem RFID-Codeträger ausgestatteten Patienten erkannt wird.

2. Vorrichtung nach Anspruch 1, **dadurch gekennzeichnet, dass** die Speichereinrichtung einen zweiten Datenspeicher enthält, der nicht als Ringspeicher ausgebildet ist.

3. Vorrichtung nach Anspruch 2, **dadurch gekennzeichnet, dass** der Benutzer die Übernahme von Daten in den ersten oder in den zweiten Datenspeicher steuern kann.

4. Vorrichtung nach einem der Ansprüche 2 bis 3, **dadurch gekennzeichnet, dass** die Übernahme von Daten in den ersten oder in den zweiten Datenspeicher zumindest teilweise aufgrund von vorbestimmten Kriterien erfolgt, die nicht vom Benutzer beeinflussbar sind.

5. Vorrichtung nach einem der Ansprüche 1 bis 4, **dadurch gekennzeichnet, dass** die Daten Informationen über bei einer chirurgischen Operation verwendete Instrumente und/oder Geräte enthalten.

6. Vorrichtung nach Anspruch 5, **dadurch gekennzeichnet, dass** die Daten Informationen über Ort und/oder Funktion und/oder Parameter der bei der chirurgischen Operation verwendeten Instrumente und/oder Geräte enthalten.

7. Vorrichtung nach einem der Ansprüche 1 bis 6, **dadurch gekennzeichnet, dass** die Daten automatisch übernommen werden.

8. Vorrichtung nach einem der Ansprüche 1 bis 7, **dadurch gekennzeichnet, dass** die Daten zumindest teilweise aus einem Netzwerk, von einem Computersystem und/oder von einem medizinischen Gerät, insbesondere einer endoskopischen Videokamera, übernommen werden.

9. Vorrichtung nach einem der Ansprüche 1 bis 8, **dadurch gekennzeichnet, dass** die Vorrichtung zur Integration in ein busgesteuertes Kommunikationssystem innerhalb des Operationssaals ausgebildet ist.

10. Vorrichtung nach einem der Ansprüche 1 bis 8, **dadurch gekennzeichnet, dass** die Daten zumindest teilweise über eine drahtlose Verbindung übernommen werden.

11. Vorrichtung nach Anspruch 10, **dadurch gekennzeichnet, dass** die Vorrichtung weiterhin mindestens eine RFID-Identifikations-und/oder Ortungsvorrichtung umfasst.

12. Vorrichtung nach Anspruch 10, **dadurch gekennzeichnet, dass** ein Sensor- oder ein RFID-System vorgesehen ist zur automatischen Detektierung und/oder Identifizierung des Patienten im Operationssaal.

13. Vorrichtung nach einem der Ansprüche 9 bis 12, **dadurch gekennzeichnet, dass** die Speicherung von Daten im Ringspeicher nach dem Verlassen des Patienten automatisch beendet wird.

14. System zur Durchführung medizinischer Eingriffe, das eine Vorrichtung zur Dokumentation medizinischer Daten nach einem der vorstehenden Ansprüche umfasst, weiterhin umfassend
- eine Einrichtung zur Steuerung und/oder Überwachung mindestens eines Geräts und
- mindestens ein mit der Steuerungs- und/oder Überwachungseinrichtung verbundenes Gerät zur Verwendung bei einem medizinischen Eingriff,
- wobei die Vorrichtung zur Dokumentation medizinischer Daten mit der Steuerungs- und/oder Überwachungseinrichtung verbunden ist.

15. System nach Anspruch 14, **dadurch gekennzeichnet, dass** es weiterhin Mittel zur Identifikation und/oder Ortsbestimmung von medizinischen Instrumenten, medizinischen Geräten und/oder Personen umfasst.

16. System nach Anspruch 15, **dadurch gekennzeichnet, dass** mindestens ein Mittel zur Identifikation und/oder Ortsbestimmung von medizinischen Instrumenten, medizinischen Geräten und/oder Personen im Eingangsbereich des OP vorgesehen ist.

17. Verfahren zur Dokumentation medizinischer Daten unter Verwendung einer Vorrichtung zur Dokumentation medizinischer Daten, die
- mindestens eine Datenübernahmeeinrichtung zur Übernahme von Daten von mindestens einer Datenquelle,
- mindestens eine Speichereinrichtung zur Speicherung von Daten, die einen ersten Datenspeicher enthält, der als Ringspeicher ausgebildet ist,
- mindestens eine Anzeigeeinrichtung zur Anzeige von Daten für einen Benutzer, und
- mindestens eine Eingabeeinrichtung zur Eingabe von Daten und/oder Anweisungen durch den Benutzer umfasst,
- wobei die Daten Bildinformationen, insbesondere endoskopische Bildinformationen und/oder Sprachdaten aus einem Operationssaal enthalten,
wobei das Verfahren folgende Schritte umfasst:
- Übernahme der medizinischen Daten in die Speichereinrichtung,
- Speichern der medizinischen Daten in dem Ringspeicher, wobei für einen Benutzer ein Zugriff auf die medizinischen Daten möglich ist,
- Löschen und/oder Überschreiben der medizinischen Daten nach einer vorbestimmten Zeit,
- wobei die Speicherung der Daten im Ringspeicher durch das Vorhandensein eines Patienten im Operationssaal ausgelöst wird, wobei das Vorhandensein des Patienten im Operationssaal durch ein RFID-System detektiert wird und wobei in einem Eingangsbereich des Operationssaals hintereinander mehrere Schleusen mit jeweils einem RFID-Lesegerät installiert sind und die Bewegungsrichtung des mit einem RFID-Codeträger ausgestatteten Patienten erkannt wird.

## Claims

1. Device for documenting medical data, comprising:
- at least one data transfer apparatus for transferring data from at least one data source,
- at least one storage apparatus for storing data,
- at least one display apparatus for displaying data to a user, and
- at least one input apparatus for entering data and/or commands by the user,
- the data containing image information, in particular endoscopic image information and/or speech data from an operating theatre, **characterized in that**
- the storage apparatus contains a first data storage medium, which is embodied as a circular buffer,
- such that data stored on the circular buffer are respectively deleted and/or overwritten after a predetermined period of time,
- the storage of data in the circular buffer being triggered by the presence of a patient in the operating theatre, the presence of the patient in the operating theatre being detected by an RFID system and a plurality of gates, each with one RFID reader, being installed one behind the other in an entry region of the operating theatre and the movement direction of the patient equipped with an RFID code carrier being identified.

2. Device according to Claim 1, **characterized in that** the storage apparatus contains a second data storage medium, which is not embodied as circular buffer.

3. Device according to Claim 2, **characterized in that** the user can control the transfer of data into the first or into the second data storage medium.

4. Device according to either of Claims 2 and 3, **characterized in that** the transfer of data into the first or into the second data storage medium is brought about, at least in part, on the basis of predetermined criteria, which cannot be influenced by the user.

5. Device according to one of Claims 1 to 4, **characterized in that** the data contain information about instruments and/or appliances used during a surgical operation.

6. Device according to Claim 5, **characterized in that** the data contain information about location and/or function and/or parameters of the instruments and/or appliances used during the surgical operation.

7. Device according to one of Claims 1 to 6, **characterized in that** the data are transferred automatically.

8. Device according to one of Claims 1 to 7, **characterized in that** the data are transferred, at least in part, from a network, from a computer system and/or from a medical appliance, in particular from an endoscopic video camera.

9. Device according to one of Claims 1 to 8, **characterized in that** the device is embodied for integration into a bus-controlled communication system within the operating theatre.

10. Device according to one of Claims 1 to 8, **characterized in that** the data are transferred, at least in part, by means of a wireless connection.

11. Device according to Claim 10, **characterized in that** the device furthermore comprises at least one RFID identification device and/or locating device.

12. Device according to Claim 10, **characterized in that** provision is made for a sensor system or an RFID system for automatically detecting and/or identifying the patient in the operating theatre.

13. Device according to one of Claims 9 to 12, **characterized in that** the storage of data in the circular buffer is automatically terminated after the patient has left.

14. System for carrying out medical interventions, comprising a device for documenting medical data according to one of the preceding claims, furthermore comprising
- an apparatus for controlling and/or monitoring at least one appliance and
- at least one appliance connected to the control and/or monitoring apparatus, for use in a medical intervention,
- the device for documenting medical data being connected to the control and/or monitoring apparatus.

15. System according to Claim 14, **characterized in that** it furthermore comprises means for identifying and/or determining the location of medical instruments, medical appliances and/or persons.

16. System according to Claim 15, **characterized in that** at least one means for identifying and/or determining the location of medical instruments, medical appliances and/or persons is provided in the entry region of the operating theatre.

17. Method for documenting medical data using a device for documenting medical data, which comprises
- at least one data transfer apparatus for transferring data from at least one data source,
- at least one storage apparatus for storing data, containing a first data storage medium embodied as circular buffer,
- at least one display apparatus for displaying data to a user, and
- at least one input apparatus for entering data and/or commands by the user,
- the data containing image information, in particular endoscopic image information and/or speech data from an operating theatre,
the method comprising the following steps:
- transferring the medical data into the storage apparatus,
- storing the medical data in the circular buffer, with access to the medical data being possible for a user,
- deleting and/or overwriting the medical data after a predetermined period of time,
- the storage of the data in the circular buffer being triggered by the presence of a patient in the operating theatre, the presence of the patient in the operating theatre being detected by an RFID system and a plurality of gates, each with one RFID reader, being installed one behind the other in an entry region of the operating theatre and the movement direction of the patient equipped with an RFID code carrier being identified.

## Revendications

1. Dispositif de documentation de données médicales comprenant :
- au moins un dispositif de prise en charge de données pour la prise en charge de données provenant d'au moins une source de données,
- au moins un dispositif d'enregistrement pour l'enregistrement des données,
- au moins un dispositif d'affichage pour l'affichage des données à l'attention d'un utilisateur, et
- au moins un dispositif de saisie pour la saisie de données et/ou d'instructions par l'utilisateur,
- les données contenant des informations d'image, notamment des informations d'image endoscopiques et/ou des données vocales provenant d'une salle d'opération, **caractérisé en ce que**
- le dispositif d'enregistrement contient une première mémoire de données qui est configurée sous la forme d'une mémoire circulaire,
- de sorte que les données enregistrées sur la mémoire circulaire sont respectivement effacées et/ou écrasées après une durée prédéfinie,
- l'enregistrement des données dans la mémoire circulaire étant déclenché par la présence d'un patient dans la salle d'opération, la présence du patient dans la salle d'opération étant détectée par un système RFID et plusieurs sas de transfert chacun équipés d'un lecteur RFID étant installés les uns derrière les autres dans une zone d'entrée de la salle d'opération et le sens de déplacement du patient équipé d'un support de code RFID étant reconnu.

2. Dispositif selon la revendication 1, **caractérisé en ce que** le dispositif d'enregistrement contient une deuxième mémoire de données qui n'est pas configurée sous la forme d'une mémoire circulaire.

3. Dispositif selon la revendication 2, **caractérisé en ce que** l'utilisateur peut commander la prise en charge des données dans la première ou dans la deuxième mémoire de données.

4. Dispositif selon l'une des revendications 2 à 3, **caractérisé en ce que** la prise en charge des données dans la première ou dans la deuxième mémoire de données s'effectue au moins partiellement sur la base de critères prédéfinis qui ne peuvent pas être influencés par l'utilisateur.

5. Dispositif selon l'une des revendications 1 à 4, **caractérisé en ce que** les données contiennent des informations sur les instruments et/ou les appareils utilisés lors d'une opération chirurgicale.

6. Dispositif selon la revendication 5, **caractérisé en ce que** les données contiennent des informations sur le lieu et/ou la fonction et/ou les paramètres des instruments et/ou des appareils utilisés lors de l'opération chirurgicale.

7. Dispositif selon l'une des revendications 1 à 6, **caractérisé en ce que** les données sont prises en charge automatiquement.

8. Dispositif selon l'une des revendications 1 à 7, **caractérisé en ce que** les données sont au moins partiellement prises en charge depuis un réseau, d'un système informatique et/ou d'un appareil médical, notamment d'une caméra vidéo endoscopique.

9. Dispositif selon l'une des revendications 1 à 8, **caractérisé en ce que** le dispositif est configuré pour être intégré dans un système de communication commandé par bus à l'intérieur de la salle d'opération.

10. Dispositif selon l'une des revendications 1 à 8, **caractérisé en ce que** les données sont au moins partiellement prises en charge par le biais d'une liaison sans fil.

11. Dispositif selon la revendication 10, **caractérisé en ce que** le dispositif comprend en outre au moins un dispositif d'identification RFID et/ou de localisation.

12. Dispositif selon la revendication 10, **caractérisé en ce qu'**il est prévu un système de capteur ou un système à RFID pour la détection et/ou l'authentification automatique du patient dans la salle d'opération.

13. Dispositif selon l'une des revendications 9 à 12, **caractérisé en ce que** l'enregistrement des données dans la mémoire circulaire prend automatiquement fin après la sortie du patient.

14. Système pour réaliser des interventions médicales, lequel comprend un dispositif de documentation de données médicales selon l'une des revendications précédentes, comprenant en outre
- un équipement pour commander et/ou surveiller au moins un appareil et
- au moins un appareil relié avec l'équipement de commande et/ou de surveillance à utiliser lors d'une intervention médicale,
- le dispositif de documentation de données médicales étant relié avec l'équipement de commande et/ou de surveillance.

15. Système selon la revendication 14, **caractérisé en ce qu'**il comprend en plus des moyens pour l'identification et/ou la détermination du lieu d'instruments médicaux, d'appareils médicaux et/ou de personnes.

16. Système selon la revendication 15, **caractérisé en ce qu'**il est prévu au moins un moyen pour l'identification et/ou la détermination du lieu d'instruments médicaux, d'appareils médicaux et/ou de personnes dans la zone d'entrée de la salle d'opération.

17. Procédé de documentation de données médicales en utilisant un dispositif de documentation de données médicales comprenant
- au moins un dispositif de prise en charge de données pour la prise en charge de données provenant d'au moins une source de données,
- au moins un dispositif d'enregistrement pour l'enregistrement des données, lequel contient une première mémoire de données qui est configurée sous la forme d'une mémoire circulaire,
- au moins un dispositif d'affichage pour l'affichage des données à l'attention d'un utilisateur, et
- au moins un dispositif de saisie pour la saisie de données et/ou d'instructions par l'utilisateur,
- les données contenant des informations d'image, notamment des informations d'image endoscopiques et/ou des données vocales provenant d'une salle d'opération, le procédé comprenant les étapes suivantes :
- prise en charge des données médicales dans le dispositif d'enregistrement,
- enregistrement des données médicales dans la mémoire circulaire, un accès aux données médicales étant possible pour un utilisateur,
- effacement et/ou écrasement des données médicales après une durée prédéfinie,
- l'enregistrement des données dans la mémoire circulaire étant déclenché par la présence d'un patient dans la salle d'opération, la présence du patient dans la salle d'opération étant détectée par un système RFID et plusieurs sas de transfert chacun équipés d'un lecteur RFID étant installés les uns derrière les autres dans une zone d'entrée de la salle d'opération et le sens de déplacement du patient équipé d'un support de code RFID étant reconnu.
